# EUROPÄISCHE PATENTSCHRIFT

(11) **EP 2 866 677 B1**
(45) Veröffentlichungstag und Bekanntmachung des Hinweises auf die Patenterteilung: **06.07.2016**
(21) Anmeldenummer: 13759438.8
(22) Anmeldetag: 17.08.2013
(51) Int. Cl.: A61B 17/00, A61B 17/3207, A61B 17/22, A61B 17/32

(54) **CHIRURGISCHES INSTRUMENT ZUR BEHANDLUNG VON FISTELN**
SURGICAL INSTRUMENT FOR THE TREATMENT OF FISTULAE
INSTRUMENT CHIRURGICAL POUR LE TRAITEMENT DE FISTULES

(30) Priorität: 18.08.2012 DE 102012016439
(43) Veröffentlichungstag der Anmeldung: 06.05.2015
(73) Patentinhaber: Lang, Reinhold, 82064 Straßlach (DE)
(72) Erfinder: Lang, Reinhold, 82064 Straßlach (DE)
(86) Internationale Anmeldenummer: PCT/EP2013/002483
(87) Internationale Veröffentlichungsnummer: WO 2014/029486

(56) Entgegenhaltungen:
- EP-A1- 1 057 457
- EP-A2- 0 136 079
- EP-A2- 1 764 046
- WO-A1-2007/072043
- WO-A2-2006/117783
- GB-A- 2 450 411
- US-A1- 2011 105 944
- US-A1- 2011 282 337
- US-A1- 2011 282 354
- US-B1- 8 105 335

## Beschreibung

Die vorliegende Erfindung betrifft ein chirurgisches Instrument zur Behandlung von Fisteln.

Fisteln sind röhrenförmige Verbindungen zwischen zwei inneren Hohlorganen eines Säugetiers oder zwischen einem inneren Hohlorgan und einer Körperoberfläche eines Säugetiers. Dabei ist für Fisteln kennzeichnend, dass diese Verbindungen nicht natürlich vorbestehen, sondern sich krankhaft beispielsweise infolge einer Entzündung oder einer mechanischen Beschädigung des Körpers bilden.

Die durch Fisteln erstellten Verbindungen sind unerwünscht. Häufig ist es daher erforderlich, Fisteln operativ zu entfernen. Hierfür werden die Fisteln entweder aufgeschnitten, insgesamt entfernt, oder gereinigt und verschlossen.

Die Reinigung von Fisteln gestaltet sich häufig schwierig, da große Mengen an infektiösem Material abtransportiert werden müssen und Fisteln häufig verwinkelt verlaufen.

Bei mit der Körperoberfläche in Verbindung stehenden rektalen oder rektovaginalen Fisteln ist es bekannt, die Fistel mittels einer zwischen zwei Fäden angeordneten Bürste zu reinigen und die Fistel anschließend mittels eines Pfropfens zu verschließen. Bei Verwendung dieser zwischen zwei Fäden angeordneten Bürste ist es nachteilig, dass zunächst ein Ende eines Fadens durch die Fistel hindurchgefädelt werden muss, was sich oft als schwierig erweist. Zudem sind die Fäden mit der dazwischen angeordneten Bürste im Einsatz insgesamt U-förmig angeordnet. Wird nun abwechselnd an einem der beiden Fäden gezogen, um die Bürste zu bewegen und die Fistel so zu reinigen, wird das radial innen in der U-Form liegende Gewebe stark belastet. Im widrigsten Fall können die Fäden in das Gewebe einschneiden.

Dokument GB 2 450 411 A offenbart ein chirurgisches Gerät mit den Merkmalen entsprechend dem Oberbegriff des Anspruchs 1, es handelt sich um ein rotierendes chirurgisches Gerät zum Reinigen von Fisteln, welches aus gehärtetem Edelstahl gebildet ist.

Ausführungsformen sind auf ein chirurgisches Gerät zur Behandlung von Fisteln gerichtet, welches leicht in die Fistel eingeführt werden kann, eine Beschädigung von Gewebe vermeidet und gleichzeitig eine zuverlässige Reinigung der Fistel erlaubt.

Die dem Anmeldungsgegenstand zugrundeliegende Aufgabe wird durch ein chirurgisches Gerät mit den im Anspruch 1 angegebenen Merkmalen gelöst. Besondere Ausführungsformen sind in den Unteransprüchen 2- 15 angegeben.

Ausführungsformen eines chirurgischen Instruments zur Behandlung von Fisteln weisen einen langgestreckten geraden Griffabschnitt und einen langgestreckten geraden Werkzeugabschnitt auf. Der Werkzeugabschnitt ist mit dem Griffabschnitt verbunden und weist an seinem dem Griffabschnitt abgewandten Ende einen Arbeitsbereich auf, an welchem eine Mehrzahl von Raspelzähnen ausgebildet sind, deren Zahnbrüste dem Griffabschnitt zugewandt sind. Der Werkzeugabschnitt weist außerhalb des Arbeitsbereichs einen maximalen Außendurchmesser von weniger als 10 mm auf.

Soweit im vorliegenden Dokument die Geometrie der Raspelzähne beschrieben wird, wird die bei Zähnen von Sägen übliche Nomenklatur verwendet.

Der Werkzeugabschnitt mit dem Arbeitsbereich kann mittels des Griffabschnitts gut in eine Fistel eingeführt werden. Mittels der Raspeln kann in den Fisteln befindliches infektiöses Material gelöst und entfernt werden. Dabei können die Raspeln durch reziproke Bewegung des Griffabschnitts hin und herbewegt werden.

Gemäß der erfindungsgemäßen Ausführungsform umgeben die Raspelzähne den Werkzeugabschnitt im Arbeitsbereich in Umfangsrichtung vollständig. Auf diese Weise wird ein gleichmäßiger Materialabtrag über die ganze Innenseite der Fistel sichergestellt.

Gemäß der erfindungsgemäßen Ausführungsform weisen die Raspelzähne quer zum Werkzeugabschnitt einen Querschnitt auf, der größer als der Querschnitt des Werkzeugabschnittes außerhalb des Arbeitsbereichs ist. Dieser Querschnitt der Raspelzähne ist gemäß der erfindungsgemäßen Ausführungsform kreisförmig. Auf diese Weise kann der Werkzeugabschnitt außerhalb des Arbeitsbereichs mit wenig Kontakt zur Innenwand der Fistel bewegt werden.

Gemäß einer Ausführungsform weist jeder Raspelzahn die Form eines Kegelstumpfes auf, und sind die Raspelzähne koaxial angeordnet, wobei die Grundflächen der Raspeln zum Griffabschnitt hin orientiert sind.

Gemäß einer alternativen Ausführungsform wechseln sich in Umfangsrichtung des Werkzeugabschnitts im Arbeitsbereich Bereiche, an denen Raspelzähne angeordnet sind, und Bereiche, die frei von Raspelzähnen sind, ab. Dann kann über die Bereiche, die frei von Raspelzähnen sind, gelöstes Material beispielsweise durch Spülen abtransportiert werden.

Gemäß einer Ausführungsform sind die Raspelzähne im Arbeitsbereich in Längsrichtung des Werkzeugabschnitts benachbart angeordnet. Gemäß einer Ausführungsform sind im Arbeitsbereich zwischen 5 und 20 und weiter insbesondere zwischen 8 und 16 Raspelzähne in Längsrichtung des Werkzeugabschnitts benachbart angeordnet.

Gemäß einer Ausführungsform weist der Arbeitsbereich eine Länge auf, die zwischen 4 % und 20 % und insbesondere zwischen 7 % und 15 % und weiter insbesondere 9 % der Länge des Werkzeugabschnitts beträgt.

Gemäß einer Ausführungsform weisen die Raspelzähne einen Schnittwinkel von zwischen 120° und 60° und insbesondere von zwischen 110° und 80° und weiter insbesondere von 90° auf.

Gemäß einer Ausführungsform weisen die Raspelzähne einen Freiwinkel von zwischen 15° und 45° und insbesondere von zwischen 20° und 40° und weiter insbesondere von 30° auf.

Gemäß einer Ausführungsform weisen die Raspelzähne eine Zahnteilung von zwischen 0,8 mm und 5,0 mm und insbesondere von zwischen 0,8 mm und 2,0 mm und insbesondere von zwischen 1,0 mm und 1,8 mm und weiter insbesondere von zwischen 1,3 mm und 3,0 mm und weiter insbesondere von 1,4 mm auf.

Gemäß einer Ausführungsform weisen die Raspelzähne eine Zahnhöhe von zwischen 0,2 mm und 2,0 mm und insbesondere von zwischen 0,2 mm und 0,8 mm und insbesondere von zwischen 0,4 mm und 0,6 mm und weiter insbesondere von 0,5 mm auf.

Gemäß einer Ausführungsform weist der Arbeitsbereich an seinem dem Griffabschnitt abgewandten Ende die Form einer Kugelkalotte auf, deren Krümmung stetig in einen Zahnrücken des benachbarten Raspelzahns übergeht.

Gemäß einer Ausführungsform weist der Werkzeugabschnitt an seinem dem Griffabschnitt abgewandten Ende des Arbeitsbereichs einen Haken auf, dessen Öffnung dem Griffabschnitt zugewandt ist. Dieser Haken kann separat zu den Raspelzähnen vorgesehen sein, oder in Form einer keilförmigen Ausnehmung in einen Raspelzahn integriert sein. An diesem Haken kann ein Fistelpfropfen befestigt und mittels des Instruments in einer zu behandelnden Fistel angeordnet werden.

Gemäß einer Ausführungsform weist der Griffabschnitt einen maximalen Außendurchmesser von mehr als 2,5 mm und insbesondere von mehr als 4 mm und weiter insbesondere von mehr als 5 mm auf und weist der Griffabschnitt gleichzeitig einen maximalen Außendurchmesser von weniger als 15 mm und insbesondere von weniger als 10 mm und weiter insbesondere von weniger als 6 mm auf. Dieser maximale Außendurchmesser kann insbesondere über die ganze Länge des Griffabschnitts konstant sein.

Gemäß einer Ausführungsform weist der Griffabschnitt eine Länge von mehr als 50 mm und insbesondere eine Länge von mehr als 80 mm und weiter insbesondere eine Länge von mehr als 100 mm auf, und weist der Griffabschnitt gleichzeitig eine Länge von weniger als 250 mm und insbesondere eine Länge von weniger als 200 mm und weiter insbesondere eine Länge von weniger als 150 mm auf.

Gemäß einer Ausführungsform weist der Werkzeugabschnitt innerhalb des Arbeitsbereichs einen maximalen Außendurchmesser von wenigstens 1,0 mm und insbesondere von wenigstens 1,5 mm und weiter insbesondere von wenigstens 2,5 mm auf, und weist der Werkzeugabschnitt gleichzeitig innerhalb des Arbeitsbereichs einen maximalen Außendurchmesser von weniger als 10,0 mm und insbesondere von weniger als 6,0 mm und weiter insbesondere von höchstens 4,5 mm auf. Dieser maximale Außendurchmesser kann insbesondere über die ganze Länge des Arbeitsbereichs des Werkzeugabschnitts konstant sein.

Gemäß einer Ausführungsform weist der Werkzeugabschnitt außerhalb des Arbeitsbereichs einen maximalen Außendurchmesser von mehr als 1,0 mm und insbesondere von mehr als 1,25 mm und weiter insbesondere von wenigstens 1,5 mm auf, und weist der Werkzeugabschnitt gleichzeitig außerhalb des Arbeitsbereichs einen maximalen Außendurchmesser von weniger als 5 mm und insbesondere von weniger als 3 mm und weiter insbesondere von höchstens 2,5 mm auf. Dieser maximale Außendurchmesser kann insbesondere über die ganze Länge des Werkzeugabschnitts außerhalb des Arbeitsbereichs konstant sein.

Gemäß einer Ausführungsform weist der Werkzeugabschnitt eine Länge von mehr als 50 mm und insbesondere eine Länge von mehr als 80 mm und weiter insbesondere eine Länge von wenigstens 100 mm auf, und weist der Werkzeugabschnitt gleichzeitig eine Länge von weniger als 250 mm und insbesondere eine Länge von weniger als 200 mm und weiter insbesondere eine Länge von weniger als 150 mm auf.

Gemäß einer Ausführungsform weist der Griffabschnitt einen Querschnitt auf, der die Form eines regelmäßigen Polygons mit drei, vier, fünf, sechs, sieben oder acht Ecken aufweist.

Gemäß einer Ausführungsform weist der Griffabschnitt einen Querschnitt auf, der sich aus drei, vier, fünf, sechs, sieben oder acht mit gleichen Winkelabständen um ein Zentrum herum angeordneten Rechtecken mit abgerundeten Ecken mit gleichen Dimensionen zusammensetzt. Gemäß einer Ausführungsform ist der Querschnitt des Griffabschnitts kreuzförmig. Griffabschnitte mit derartigen Querschnitten weisen eine erhöhe Biegesteifigkeit bei minimalem Materialeinsatz auf, und lassen sich durch einen Benutzer auch mit Handschuhen drehfest greifen.

Gemäß einer Ausführungsform weisen die Raspelzähne wenigstens eine Nut auf, die sich gerade über die ganze Längserstreckung des Arbeitsbereichs erstreckt oder die sich spiralig über die ganze Längserstreckung und den ganzen Umfang des Arbeitsbereichs erstreckt. Über eine derartige Nut kann beispielsweise ein Spülen erfolgen und der Materialabtrag verbessert werden. Weiter erlaubt diese Nut den Eintrag von Medikamenten.

Gemäß einer Ausführungsform sind im Arbeitsbereich abwechselnd Raspelzähne mit unterschiedlicher Geometrie und insbesondere mit unterschiedlicher Zahnteilung angeordnet. Beispielsweise können Raspelzähne für den Materialabtrag und Raspelzähne für den Materialtransport vorgesehen sein.

Gemäß der erfindungsgemäßen Ausführungsform ist das chirurgische Instrument einstückig aus Kunststoff und insbesondere aus thermoplastischem Kunststoff und weiter insbesondere aus Polyetheretherketon gebildet.

Gemäß einer Ausführungsform ist das chirurgische Instrument einstückig aus einem Material gebildet, dessen Rockwellhärte nach ISO 868 und ISO 2039-2 zwischen M90 und M108 und insbesondere zwischen M95 und M103 und weiter insbesondere M99 beträgt.

Gemäß einer Ausführungsform ist das chirurgische Instrument einstückig aus einem Material gebildet, dessen Izod-Kerbschlagzähigkeit bei 23 °C nach ISO 180/1A zwischen 5,8 und 7,0 KJ/m2 und insbesondere zwischen 6,2 und 6,6 KJ/m2 und weiter insbesondere 6,4 KJ/m2 beträgt.

Gemäß einer Ausführungsform ist das chirurgische Instrument einstückig aus einem Material gebildet, dessen Charpy-Kerbschlagzähigkeit bei 23 °C nach ISO 179/1eA zwischen 7,8 und 8,6 und insbesondere zwischen 8,0 und 8,4 KJ/m2 und weiter insbesondere 8,2 KJ/m2 beträgt.

Gemäß einer Ausführungsform sind der Griffabschnitt und der Werkzeugabschnitt entlang einer Geraden angeordnet.

Gemäß einer Ausführungsform weist der Griffabschnitt einen größeren maximalen Durchmesser auf, als der Werkzeugabschnitt.

In diesem Zusammenhang wird darauf hingewiesen, dass die in dieser Beschreibung und den Ansprüchen zur Aufzählung von Merkmalen verwendeten Begriffe "umfassen", "aufweisen", "beinhalten", "enthalten" und "mit", sowie deren grammatikalische Abwandlungen, generell als nichtabschließende Aufzählung von Merkmalen, wie z. B. Verfahrensschritten, Einrichtungen, Bereichen, Größen und dergleichen aufzufassen sind, und in keiner Weise das Vorhandensein anderer oder zusätzlicher Merkmale oder Gruppierungen von anderen oder zusätzlichen Merkmalen ausschließen.

Weitere Merkmale der Erfindung ergeben sich aus der nachfolgenden Beschreibung von Ausführungsbeispielen in Verbindung mit den Ansprüchen sowie den Figuren. In den Figuren werden gleiche bzw. ähnliche Elemente mit gleichen bzw. ähnlichen Bezugszeichen bezeichnet. Es wird darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung eines Ausführungsbeispiels der Erfindung wird auf die beiliegenden Figuren Bezug genommen, von denen
- Figur 1: unterschiedliche Ansichten auf ein chirurgisches Instrument zur Behandlung von Fisteln gemäß einer erfindungsgemäßen Ausführungsform zeigt;
- Figur 2: unterschiedliche Ansichten auf ein chirurgisches Instrument zur Behandlung von Fisteln gemäß einer ersten beispielhaften Ausführungsform zeigt;
- Figur 3: unterschiedliche Ansichten auf ein chirurgisches Instrument zur Behandlung von Fisteln gemäß einer zweiten beispielhaften Ausführungsform zeigt; und
- Figur 4: unterschiedliche Ansichten auf ein chirurgisches Instrument zur Behandlung von Fisteln gemäß einer dritten beispielhaften Ausführungsform zeigt.

Die in den Figuren angegebenen Maßangaben sind nur beispielhaft. Die Einheit der Längemaße ist Millimeter.

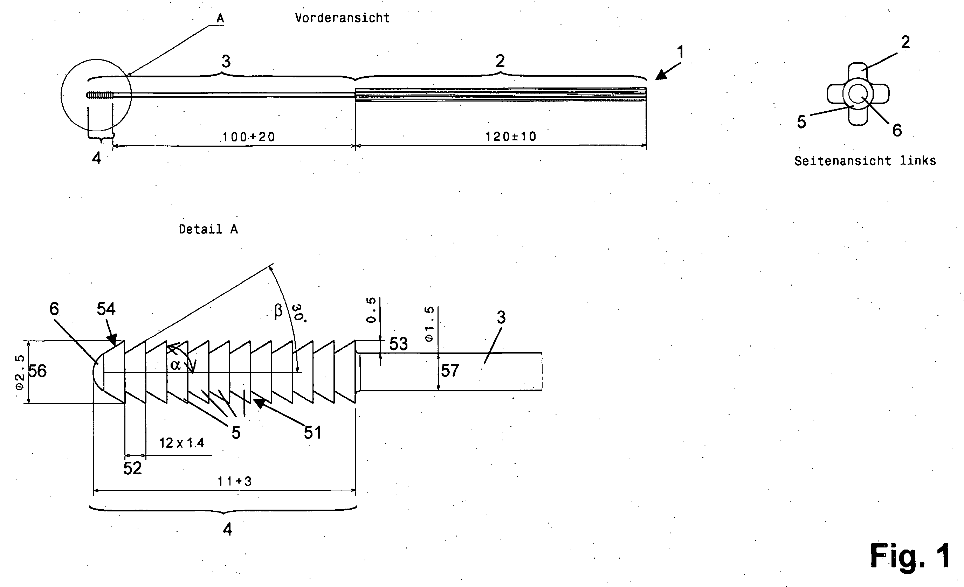

Im Folgenden wird unter Bezugnahme auf Figur 1 eine Ausführungsform eines erfindungsgemäßen chirurgischen Instruments zur Behandlung von Fisteln beschrieben.

Die Figuren 1 bis 3 zeigen jeweils links oben eine Seitenansicht auf ein chirurgisches Instrument zur Behandlung von Fisteln, links unten eine Vergrößerte Seitenansicht der Spitze im Bereich "A", und rechts oben eine koaxiale Seitenansicht von vorne auf die Spitze des Instruments.

Das in Figur 1 gezeigte chirurgische Instrument 1 ist aus einem sich geradlinig erstreckenden langgestreckten Griffabschnitt 2 und einem koaxial mit dem Griffabschnitt 2 verbundenen sich geradlinig erstreckenden langgestreckten Werkzeugabschnitt 3 gebildet. Der Griffabschnitt 2 und der Werkzeugabschnitt 3 sind einstückig miteinander verbunden und durch Spritzgießen von Polyetheretherketon gebildet.

An seinem dem Griffabschnitt 2 abgewandten Ende weist der Werkzeugabschnitt 3 einen Arbeitsbereich 4 auf, der im Detail A vergrößert dargestellt ist.

Ersichtlich sind an dem Werkzeugabschnitt 3 im Arbeitsbereich 4 eine Mehrzahl von Raspelzähnen 5 ausgebildet und einstückig mit dem Werkzeugabschnitt 3 verbunden.

In der gezeigten Ausführungsform weist jeder Raspelzahn 5 die Form eines Kegelstumpfes auf. Die Raspelzähne 5 sind koaxial angeordnet. Die Grundflächen der Raspelzähne 5 sind zum Griffabschnitt 2 hin orientiert. Damit sind die Zahnbrüste 51 der Raspelzähne 5 dem Griffabschnitt 2 zugewandt. Die Arbeitsrichtung des Instruments, bei deren Bewegung der größte Materialabtrag erfolgt, ist somit ein Ziehen des Instruments am Griffabschnitt 2 in die von dem Werkzeugabschnitt 3 wegweisende Richtung.

Aufgrund ihrer kegelstrumpfförmigen Ausgestaltung umgeben die Raspelzähne 5 den Werkzeugabschnitt 3. im Arbeitsbereich 4 in Umfangsrichtung vollständig und weisen einen kreisförmigen Querschnitt auf, der größer als der Querschnitt des Werkzeugabschnittes 3 außerhalb des Arbeitsbereichs 4 ist, und maximal 2,5 mm beträgt.

In der gezeigten Ausführungsform sind im Arbeitsbereich 4 in Längsrichtung des Werkzeugabschnitts 3 zwölf Raspelzähne 5 zueinander benachbart angeordnet. Auch wenn die Raspelzähne 5 in der gezeigten Ausführungsform unmittelbar aneinander angrenzen, kann zwischen den einzelnen Raspelzähnen 5 in Längsrichtung des Werkzeugabschnitts 3 im Arbeitsbereich 4 auch ein kleiner Abstand vorgesehen sein, der frei von Raspelzähnen 5 ist. Natürlich ist die vorliegende Erfindung nicht auf die Verwendung von genau zwölf Raspelzähnen beschränkt.

In der gezeigten Ausführungsform weisen die Raspelzähne 5 einen Schnittwinkel α von 90° und einen Freiwinkel β von 30° auf. Weiter weisen die Raspelzähne 5 in der gezeigten Ausführungsform eine Zahnteilung 52 von 1,4 mm und eine Zahnhöhe 53 von 0,5 mm auf. Es wird betont, dass die vorliegende Erfindung nicht auf diese Dimensionierung der Geometrie der Raspelzähne beschränkt ist.

An seinem dem Griffabschnitt 2 abgewandten Ende weist der Arbeitsbereich 4 die Form einer Kugelkalotte 6 auf, deren Krümmung stetig in den Zahnrücken 54 des benachbarten Raspelzahns 5 übergeht.

Wie gut aus der koaxialen Seitenansicht von vorne auf die Spitze des Instruments rechts oben in Figur 1 ersichtlich, weist der Griffabschnitt 2 einen Querschnitt auf, der sich aus vier mit gleichen Winkelabständen um ein Zentrum herum angeordneten Rechtecken mit abgerundeten Ecken mit gleichen Dimensionen zusammensetzt und so einen kreuzförmigen Querschnitt bildet.

In der gezeigten Ausführungsform beträgt eine Länge des Griffabschnitts 120 mm ± 10 mm und eine Länge des Werkzeugabschnitts 111 mm + 23 mm. Die Länge des Arbeitsbereichs 4 beträgt 11 mm + 3 mm. Somit beträgt die Länge des Arbeitsbereichs 4 etwa 9 % der Länge des Werkzeugabschnitts 3.

Weiter weist der Griffabschnitt 2 in der gezeigten Ausführungsform einen maximalen Außendurchmesser von 5,5 mm auf.

Außerhalb des Arbeitsbereichs 4 weist der Werkzeugabschnitt 3 in der gezeigten Ausführungsform einen konstanten Außendurchmesser 57 von 1,5 mm auf.

Innerhalb des Arbeitsbereichs 4 weist der Werkzeugabschnitt 3 in der gezeigten Ausführungsform einen konstanten Außendurchmesser 56 von 2,5 mm auf.

Es wird betont, dass von der vorstehenden Geometrie des Griffabschnitts 2 und des Werkzeugabschnitts 3 auch abgewichen werden kann.

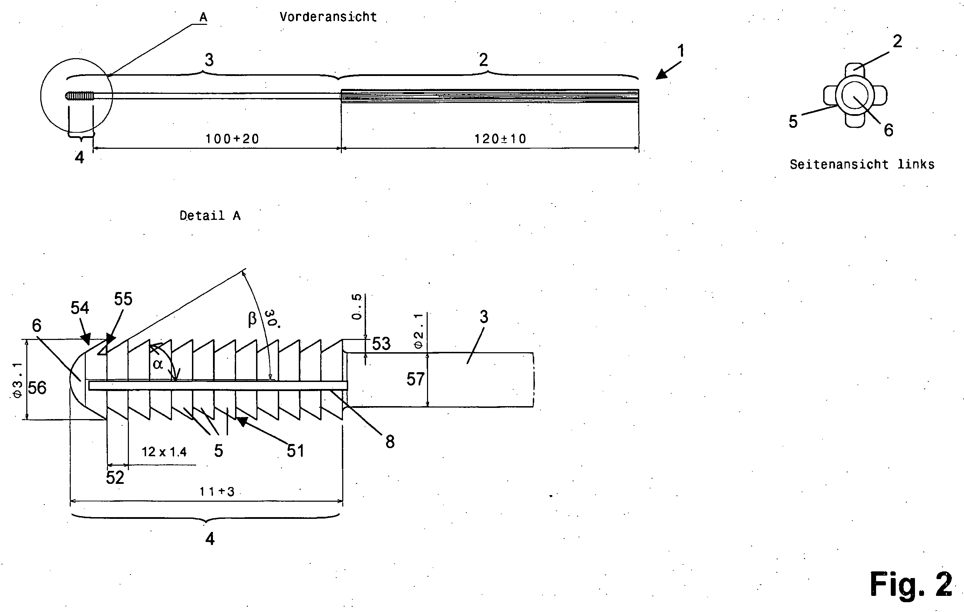

Im Folgenden wird unter Bezugnahme auf Figur 2 eine erste beispielhafte Ausführungsform eines chirurgischen Instruments zur Behandlung von Fisteln beschrieben. Dabei werden insbesondere die Unterschiede zu der vorstehenden erfindungsgemäßen Ausführungsform beschrieben. Ansonsten wird auf die erfindungsgemäßen Ausführungsform Bezug genommen.

Das chirurgische Instrument gemäß der in Figur 2 gezeigten ersten beispielhaften Ausführungsform unterscheidet sich von der vorstehend beschriebenen erfindungsgemäßen Ausführungsform insbesondere dadurch, dass der konstante Außendurchmesser 57 des Werkzeugabschnitts 3 außerhalb des Arbeitsbereichs 4 gegenüber der erfindungsgemäßen Ausführungsform erhöht ist und 2,1 mm beträgt. Entsprechend ist auch der maximale Durchmesser 56 der Raspelzähne 5 gegenüber der erfindungsgemäßen Ausführungsform erhöht und beträgt 3,1 mm. Damit ist dieses chirurgische Instrument zur Behandlung von Fisteln mit größeren Innendurchmessern ausgebildet.

In dieser ersten beispielhaften Ausführungsform weisen die Raspelzähne 5 eine Nut 8 auf, die sich gerade über die ganze Längserstreckung des Arbeitsbereichs 4 erstreckt, und ein Spülen während des Materialabtrages erlaubt.

Zudem weist in der in Figur 2 gezeigten beispielhaften Ausführungsform derjenige Raspelzahl 5, welcher von dem Griffabschnitt 2 am weitesten entfernt ist, eine keilförmige Ausnehmung 55 auf, in welche beispielsweise ein Faden oder ein Fistelpfropfen eingehakt werden kann.

Es wird betont, dass von der vorstehenden Geometrie des Werkzeugabschnitts 3 auch abgewichen werden kann. Weiter sind die Nut 8 und die Ausnehmung 55 nur fakultativ.

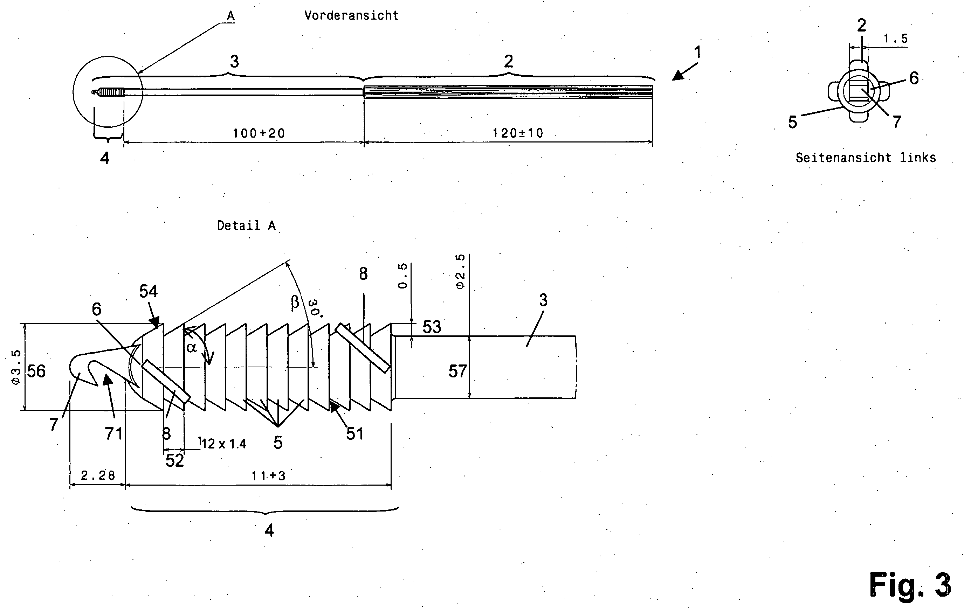

Im Folgenden wird unter Bezugnahme aüf Figur 3 eine zweite beispielhafte Ausführungsform eines chirurgischen Instruments zur Behandlung von Fisteln beschrieben. Dabei werden insbesondere die Unterschiede zu der vorstehenden erfindungsgemäßen Ausführungsform beschrieben. Ansonsten wird auf die erfindungsgemäßen Ausführungsform Bezug genommen.

Das chirurgische Instrument gemäß der in Figur 3 gezeigten beispielhaften zweiten Ausführungsform unterscheidet sich von der erfindungsgemäßen Ausführungsform insbesondere dadurch, dass der konstante Außendurchmesser 57 des Werkzeugabschnitts 3 außerhalb des Arbeitsbereichs 4 gegenüber der erfindungsgemäßen Ausführungsform weiter erhöht ist und 2,5 mm beträgt. Entsprechend ist auch der maximale Durchmesser 56 der Raspelzähne 5 gegenüber der ersten Ausführungsform weiter erhöht und beträgt 3,5 mm. Damit ist dieses chirurgische Instrument zur Behandlung von Fisteln mit besonders großen Innendurchmessern ausgebildet.

In dieser zweiten beispielhaften Ausführungsform weisen die Raspelzähne 5 eine Nut 8 auf, die sich spiralig über die ganze Längserstreckung und den ganzen Umfang des Arbeitsbereichs 4 erstreckt, und ein Spülen während des Materialabtrages erlaubt.

Weiter weist der Werkzeugabschnitt 3 an seinem dem Griffabschnitt 2 abgewandten Ende des Arbeitsbereichs 4 einen Haken 7 auf, dessen Öffnung 71 dem Griffabschnitt 2 zugewandt ist. Der Haken 7 weist einen rechteckigen Querschnitt auf, wobei die Breite 1,5 mm beträgt. Die Länge des Hakens 7 beträgt 2,28 mm. Mittels des Hakens 7 kann beispielsweise ein Fistelpfropf ergriffen und appliziert werden.

Es wird betont, dass von der vorstehenden Geometrie des Werkzeugabschnitts 3 und des Hakens 7 auch abgewichen werden kann. Weiter ist die Nut 8 nur fakultativ.

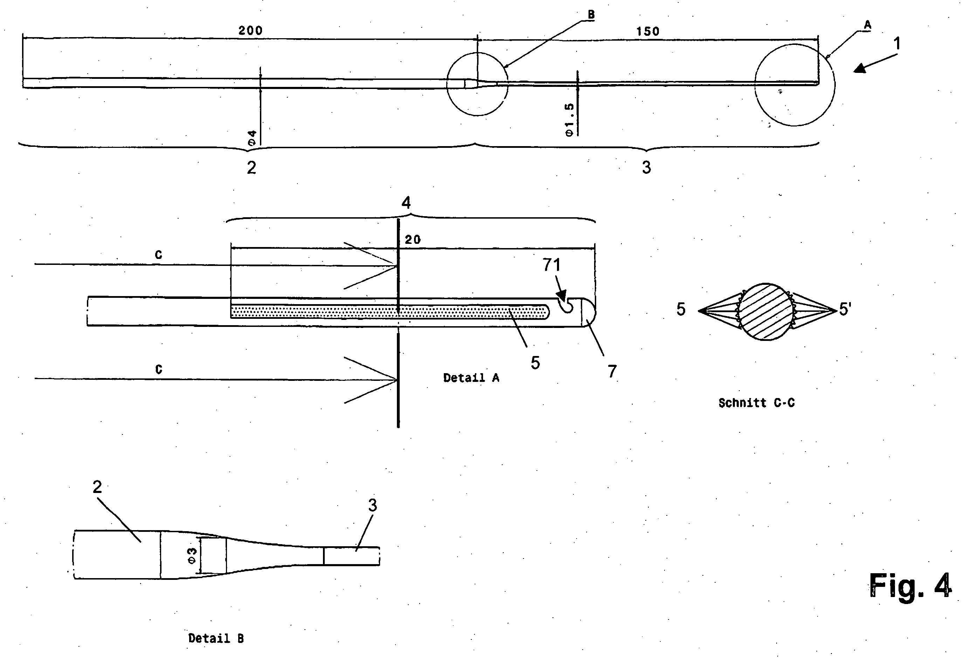

Im Folgenden wird unter Bezugnahme auf Figur 4 eine dritte beispielhafte Ausführungsform eines chirurgischen Instruments zur Behandlung von Fisteln beschrieben.

Die Figur 4 zeigt oben eine Seitenansicht auf ein chirurgisches Instrument zur Behandlung von Fisteln, in der Mitte links eine vergrößerte Seitenansicht der Spitze im Bereich "A", in der Mitte rechts einen Querschnitt durch die Spitze des Instruments entlang der Achse C-C, sowie unten links eine vergrößerte Seitenansicht des Übergangs zwischen Griffabschnitt 2 und Werkzeugabschnitt 3 im Bereich "B".

Das chirurgische Instrument 1 gemäß der dritten beispielhaften Ausführungsform ist ebenfalls einstückig aus thermoplastischem Kunststoff gebildet und weist einen zylindrischen Griffabschnitt 2 von 200 mm Länge und 4 mm Durchmesser sowie einen zylindrischen Werkzeugabschnitt 3 von 150 mm Länge und 1,5 mm Durchmesser auf. Griffabschnitt 2 und Werkzeugabschnitt 3 sind koaxial angeordnet, und weisen einen stetigen Übergang zwischen Griffabschnitt 2 und Werkzeugabschnitt 3 auf.

Im Arbeitsbereich 4 wechseln sich in Umfangsrichtung des Werkzeugabschnitts 3 Bereiche, an denen Raspelzähne 5, 5' angeordnet sind, und Bereiche, die frei von Raspelzähnen 5, 5' sind, ab. Dabei weisen die Raspelzähne 5 in einem Bereich eine Zahnhöhe 53 und Zahnteilung 52 auf, welche sich von der Zahnhöhe 53 und Zahnteilung 52 der Raspelzähne 5' in einem anderen Bereich unterscheidet.

Auch in dieser dritten beispielhaften Ausführungsform weist das chirurgische Instrument einen Haken 7 auf, dessen Öffnung hin zum Griffabschnitt 2 orientiert ist. Weiter weist auch in dieser Ausführungsform der Arbeitsbereich 4 an seinem dem Griffabschnitt 2 abgewandten Ende die Form einer Kugelkalotte auf.

Es wird betont, dass die besonderen Merkmale der vorstehenden Ausführungsformen auch miteinander kombiniert werden können. So kann beispielsweise sowohl eine keilförmige Ausnehmung 55 in einem Raspelzahn 5 als auch ein Haken 7 vorgesehen sein.

## Patentansprüche

1. Chirurgisches Instrument (1) zur Behandlung von Fisteln aufweisend:
einen langgestreckten geraden Griffabschnitt (2); und
einen langgestreckten geraden Werkzeugabschnitt (3);
wobei der Werkzeugabschnitt (3) mit dem Griffabschnitt (2) verbunden ist und an seinem dem Griffabschnitt (2) abgewandten Ende einen Arbeitsbereich (4) aufweist, an welchem eine Mehrzahl von Raspelzähnen (5) ausgebildet sind, deren Zahnbrüste (51) dem Griffabschnitt (2) zugewandt sind;
wobei der Werkzeugabschnitt (3) außerhalb des Arbeitsbereichs (4) einen maximalen Außendurchmesser (57) von weniger als 10 mm aufweist,
**dadurch gekennzeichnet,**
**dass** die Raspelzähne (5) den Werkzeugabschnitt (3) im Arbeitsbereich (4) in Umfangsrichtung vollständig umgeben;
**dass** die Raspelzähne (5) Quer zum Werkzeugabschnitt (3) einen kreisförmigen Querschnitt aufweisen, der größer als der Querschnitt des Werkzeugabschnittes außerhalb des Arbeitsbereichs (4) ist; und
**dass** das chirurgische Instrument einstückig aus Kunststoff gebildet ist.

2. Chirurgisches Instrument (1) nach Anspruch 1, wobei im Arbeitsbereich (4) zwischen 5 und 20 oder zwischen 8 und 16 Raspelzähne (5) in Längsrichtung des Werkzeugabschnitts (3) benachbart angeordnet sind.

3. Chirurgisches Instrument (1) nach Anspruch 1 oder 2, wobei der Arbeitsbereich (4) eine Länge aufweist, die zwischen 4 % und 20 % oder zwischen 7 % und 15 % oder 9 % der Länge des Werkzeugabschnitts (3) beträgt.

4. Chirurgisches Instrument (1) nach einem der Ansprüche 1 bis 3, wobei die Raspelzähne (5) einen Schnittwinkel (α) von zwischen 120° und 60° oder von zwischen 110° und 80° oder von 90° aufweisen.

5. Chirurgisches Instrument (1) nach einem der Ansprüche 1 bis 4, wobei die Raspelzähne (5) einen Freiwinkel (β) von zwischen 15° und 45° oder von zwischen 20° und 40° oder von 30° aufweisen.

6. Chirurgisches Instrument (1) nach einem der Ansprüche 1 bis 5, wobei die Raspelzähne (5) eine Zahnteilung (52) von zwischen 0,8 mm und 5,0 mm oder von zwischen 0,8 mm und 2,0 mm oder von zwischen 1,0 mm und 1,8 mm oder von zwischen 1,3 mm und 3,0 mm oder von 1,4 mm aufweisen.

7. Chirurgisches Instrument (1) nach einem der Ansprüche 1 bis 6, wobei die Raspelzähne (5) eine Zahnhöhe (53) von zwischen 0,2 mm und 2,0 mm oder von zwischen 0,2 mm und 0,8 mm oder von zwischen 0,4 mm und 0,6 mm oder von 0,5 mm aufweisen.

8. Chirurgisches Instrument (1) nach einem der Ansprüche 1 bis 7, wobei der Arbeitsbereich (4) an seinem dem Griffabschnitt (2) abgewandten Ende die Form einer Kugelkalotte (6) aufweist, deren Krümmung stetig in einen Zahnrücken (54) des benachbarten Raspelzahns (5) übergeht.

9. Chirurgisches Instrument (1) nach einem der Ansprüche 1 bis 8, wobei der Werkzeugabschnitt (3) an seinem dem Griffabschnitt (2) abgewandten Ende des Arbeitsbereichs (4) einen Haken (7) aufweist, dessen Öffnung (71) dem Griffabschnitt (2) zugewandt ist.

10. Chirurgisches Instrument (1) nach einem der Ansprüche 1 bis 9, wobei der Griffabschnitt (2) einen maximalen Außendurchmesser von mehr als 2,5 mm oder von mehr als 4 mm oder von mehr als 5 mm aufweist, und der Griffabschnitt (2) gleichzeitig einen maximalen Außendurchmesser von weniger als 15 mm oder von weniger als 10 mm oder von weniger als 6 mm aufweist.

11. Chirurgisches Instrument (1) nach einem der Ansprüche 1 bis 10,
wobei der Werkzeugabschnitt (3) innerhalb des Arbeitsbereichs (4) einen maximalen Außendurchmesser (56) von wenigstens 1,0 mm oder von wenigstens 1,5 mm oder von wenigstens 2,5 mm aufweist, und der Werkzeugabschnitt (3) gleichzeitig innerhalb des Arbeitsbereichs (4) einen maximalen Außendurchmesser von weniger als 10,0 mm oder von weniger als 6,0 mm oder von höchstens 4,5 mm aufweist; und
wobei der Werkzeugabschnitt (3) außerhalb des Arbeitsbereichs (4) einen maximalen Außendurchmesser von mehr als 1,0 mm oder von mehr als 1,25 mm oder von wenigstens 1,5 mm aufweist, und der Werkzeugabschnitt (3) gleichzeitig außerhalb des Arbeitsbereichs (4) einen maximalen Außendurchmesser von weniger als 5 mm oder von weniger als 3 mm oder von höchstens 2,5 mm aufweist.

12. Chirurgisches Instrument (1) nach einem der Ansprüche 1 bis 11,
wobei der Griffabschnitt (2) einen Querschnitt aufweist, der die Form eines regelmäßigen Polygons mit drei, vier, fünf, sechs, sieben oder acht Ecken aufweist;
oder
wobei der Griffabschnitt (2) einen Querschnitt aufweist, der sich aus drei, vier, fünf, sechs, sieben oder acht mit gleichen Winkelabständen um ein Zentrum herum angeordneten Rechtecken mit abgerundeten Ecken mit gleichen Dimensionen zusammensetzt oder kreuzförmig ist.

13. Chirurgisches Instrument (1) nach einem der Ansprüche 1 bis 12, wobei im Arbeitsbereich (4) abwechselnd Raspelzähne (5, 5') mit unterschiedlicher Geometrie oder mit unterschiedlicher Zahnteilung (52) angeordnet sind.

14. Chirurgisches Instrument (1) nach einem der Ansprüche 1 bis 13, wobei das chirurgische Instrument einstückig aus thermoplastischem Kunststoff oder aus Polyetheretherketon gebildet ist.

15. Chirurgisches Instrument (1) nach einem der Ansprüche 1 bis 14, wobei jeder Raspelzahn (5) die Form eines Kegelstumpfes aufweist, und die Raspelzähne (5) koaxial angeordnet sind, wobei die Grundflächen der Raspelzähne (5) zum Griffabschnitt (2) hin orientiert sind.

## Claims

1. A surgical instrument (1) for the treatment of fistulas comprising:
an elongated straight handle portion (2); and
an elongated straight tool section (3);
wherein the tool section (3) is connected to the handle portion (2) and wherein the tool section (3) has a working area (4) at its end facing away from the grip portion (2), in which a plurality of rasp teeth (5) are provided the tooth-breasts (51) of which facing the grip portion (2);
wherein the tool section (3) has a maximum outer diameter (57) of less than 10 mm outside the working area (4);
**characterized in**
**that** the rasp teeth (5) completely surrounded the tool section (3) in circumferential direction in the working area (4); and
**that** the rasp teeth (5) have a circular cross-section in a direction transverse to the tool section (3), the circular cross-section being larger than the cross section of the tool section outside the working area (4); and
**that** the surgical instrument is integrally formed from plastics.

2. The surgical instrument (1) according to claim 1, wherein between 5 and 20 or between 8 and 16 rasp teeth (5) are provided along the longitudinal direction of the tool section (3) in the working area (4).

3. The surgical instrument (1) according to claim 1 or 2, wherein the working area (4) has a length which is between 4 % and 20 % or is between 7 % and 15 %, or 9 % of the length of the tool section (3).

4. The surgical instrument (1) according to any one of claims 1 to 3, wherein the rasp teeth (5) have a cutting angle (α) of between 120° and 60° or of between 110° and 80° or of 90 °

5. The surgical instrument (1) according to any one of claims 1 to 4, wherein the rasp teeth (5) have a relief angle (β) of between 15° and 45° or of between 20° and 40° or of 30°.

6. The surgical instrument (1) according to any one of claims 1 to 5, wherein the rasp teeth (5) have a tooth pitch (52) of between 0.8 mm and 5.0 mm or of between 0.8 mm and 2.0 mm or of between 1.0 mm and 1.8 mm or of between 1.3 mm and 3.0 mm or of 1.4 mm.

7. The surgical instrument (1) according to any one of claims 1 to 6, wherein the rasp teeth (5) have a tooth depth (53) of between 0.2 mm and 2.0 mm or of between 0.2 mm and 0.8 mm or of between 0.4 mm and 0.6 mm or of 0.5 mm.

8. The surgical instrument (1) according to one of claims 1 to 7, wherein the working area (4) has the shape of a calotte shell (6) at its end facing away from the grip portion (2), the curvature of which continuously merging into a tooth back (54) of the adjacent rasp tooth (5).

9. The surgical instrument (1) according to any one of claims 1 to 8, wherein the tool section (3) has a hook (7) at its end of the working area (4) facing away from the grip portion (2), the opening (71) of which being facing the grip portion (2).

10. The surgical instrument (1) according to one of claims 1 to 9, wherein the handle portion (2) has a maximum outside diameter of more than 2.5 mm or of more than 4 mm or of more than 5 mm, and the handle portion (2) has at the same time a maximum outer diameter of less than 15 mm or of less than 10 mm or of less than 6 mm.

11. The surgical instrument (1) according to one of claims 1 to 10,
wherein the tool section (3) has a maximum outer diameter (56) of at least 1.0 mm or of at least 1.5 mm or of at least 2.5 mm within the working area (4), and the tool section (3) has at the same time a maximum outer diameter of less than 10.0 mm or less than 6.0 mm or of at most 4.5 mm within the working area (4); and
wherein the tool section (3) outside the working area (4) has a maximum outside diameter of more than 1.0 mm or of more than 1.25 mm or of at least 1.5 mm, and the tool section (3) has at the same time outside the working area (4) a maximum outer diameter of less than 5 mm and especially less than 3 mm or of at most 2.5 mm.

12. The surgical instrument (1) according to any one of claims 1 to 11,
wherein the handle portion (2) has a cross section showing the shape of a regular polygon with three, four, five, six, seven or eight corners; or
wherein the handle portion (2) has a cross section consisting of three, four, five, six, seven or eight rectangles with rounded corners and same dimensions, the rectangles being arranged at equal angular intervals around a center having or wherein the cross section is cross-shaped.

13. The surgical instrument (1) according to one of claims 1 to 12, wherein rasp teeth (5, 5') of different geometry or with differing tooth pitch (52) are alternately arranged in the working area (4).

14. The surgical instrument (1) according to one of claims 1 to 13, wherein the surgical instrument is integrally formed of thermoplastic plastic or of polyether ether ketone.

15. A surgical instrument (1) according to any one of claims 1 to 14, wherein each rasp tooth (5) has the shape of a truncated cone, and the rasp teeth (5) being arranged coaxially, wherein the base surfaces of the rasp teeth (5) are oriented towards the handle portion (2).

## Revendications

1. Instrument chirurgical (1) pour le traitement de fistules comprenant:
une partie de poignée de forme allongée et droite (2); et
une partie d'outil de forme allongée et droite (3);
la partie de l'outil (3) étant reliée à la partie de la poignée (2) et comprenant une zone de travail (4) au niveau de son extrémité opposée à la partie de la poignée (2), la plupart des dents de râpage (5), dont la face avant des dents (51) est orientée vers la partie de la poignée (2), se trouvent dans la zone de travail;
la partie de l'outil (3) faisant état d'un diamètre extérieur maximum (57) de moins de 10 mm à l'extérieur de la zone de travail(4),
**caractérisé par**
que les dents de râpage (5) entourent entièrement la partie de l'outil (3) dans la zone de travail (4) dans la direction circonférentielle;
que les dents de râpage (5) font état d'une coupe transversale circulaire à travers la partie de l'outil (3) et cette coupe est plus grande que la coupe transversale de la partie de l'outil de la zone de travail (4); et
que l'instrument chirurgical est formé en un seul bloc d'une matière plastique.

2. Instrument chirurgical (1) selon la revendication 1, avec entre 5 et 20 ou entre 8 et 16 dents de râpage (5) agencées adjacentes les unes aux autres dans le sens longitudinal de la partie de l'outil (3) dans la zone de travail (4).

3. Instrument chirurgical (1) selon la revendication 1 ou 2, avec la zone de travail (4) étant d'une longueur comprise entre 4 % et 20 % ou entre 7 % et 15 % ou 9 % de la longueur de la partie de l'outil (3).

4. Instrument chirurgical (1) selon l'une des revendications 1 à 3, les dents de râpage (5) faisant état d'un angle de coupe (α) compris entre 120° et 60° ou entre 110° et 80° ou de 90°.

5. Instrument chirurgical (1) selon l'une des revendications 1 à 4, **caractérisé par le fait que** les dents de râpage (5) font état d'un angle de dégagement (β) compris entre 15° et 45° ou entre 20° et 40° ou de 30°.

6. Instrument chirurgical (1) selon l'une des revendications 1 à 5, les dents de râpage (5) faisant état d'une denture (52) comprise entre 0,8 mm et 5,0 mm ou entre 0,8 mm et 2,0 mm ou entre 1,0 mm et 1,8 mm ou entre 1,3 mm et 3,0 mm ou de 1,4 mm.

7. Instrument chirurgical (1) selon l'une des revendications 1 à 6, les dents de râpage (5) faisant état d'une hauteur de dent (53) comprise entre 0,2 mm et 2,0 mm ou entre 0,2 mm et 0,8 mm ou entre 0,4 mm et 0,6 mm ou de 0,5 mm.

8. Instrument chirurgical (1) selon l'une des revendications 1 à 7, la zone de travail (4) faisant état au niveau de son extrémité opposée à la partie de la poignée (2) de la forme d'une calotte sphérique (6) dont la courbure passe de manière continue au dos de dent (54) de la dent de râpage avoisinante (5).

9. Instrument chirurgical (1) selon l'une des revendications 1 à 8, la partie de l'outil (3) faisant état d'un crochet (7), au niveau de son extrémité opposée à la partie de la poignée (2) de la zone de travail (4), dont l'ouverture (71) est orientée vers la partie de la poignée (2).

10. Instrument chirurgical (1) selon l'une des revendications 1 à 9, la partie de la poignée (2) faisant état d'un diamètre extérieur maximum de plus de 2,5 mm ou de plus de 4 mm ou de plus de 5 mm, et la partie de la poignée (2) faisant en même temps état d'un diamètre extérieur maximum de moins de 15 mm ou de moins de 10 mm ou de moins de 6 mm.

11. Instrument chirurgical (1) selon l'une des revendications 1 à 10,
la partie de l'outil (3) à l'intérieur de la zone de travail (4) faisant état d'un diamètre extérieur maximum (56) d'au moins 1,0 mm ou d'au moins 1,5 mm ou d'au moins 2,5 mm, et la partie de l'outil (3) faisant en même temps état d'un diamètre extérieur maximum à l'intérieur de la zone de travail (4) de moins que 10,0 mm ou de moins que 6,0 mm ou de 4,5 mm maximum; et
la partie de l'outil (3) faisant état d'un diamètre extérieur maximum à l'extérieur de la zone de travail (4) de plus de 1,0 mm ou de plus de 1,25 mm ou d'au moins 1,5 mm, et la partie de l'outil (3) faisant en même temps état d'un diamètre extérieur maximum à l'extérieur de la zone de travail (4) de moins de 5 mm ou de moins de 3 mm ou de 2,5 mm maximum.

12. Instrument chirurgical (1) selon l'une des revendications 1 à 11,
la partie de la poignée (2) faisant état d'une coupe transversale ayant la forme d'un polygone régulier avec trois, quatre, cinq, six, sept ou huit angles;
ou
la partie de la poignée (2) faisant état d'une coupe transversale composée de trois, quatre, cinq, six, sept ou huit rectangles disposés à des écarts angulaires égaux autour d'un centre comprenant des angles arrondis aux dimensions identiques ou cruciformes.

13. Instrument chirurgical (1) selon l'une des revendications 1 à 12, les dents de râpage (5, 5') étant disposées alternativement dans des géométries différentes ou avec des dentures différentes (52) dans la zone de travail (4).

14. Instrument chirurgical (1) selon l'une des revendications 1 à 13, l'instrument chirurgical étant formé en un seul bloc d'une matière thermoplastique ou de polyétheréthercétone.

15. Instrument chirurgical (1) selon l'une des revendications 1 à 14, chaque dent de râpage (5) ayant la forme d'un tronc de cône, et les dents de râpage (5) étant disposées coaxialement, les surfaces de base des dents de râpage (5) sont orientées vers la partie de la poignée (2).
